# EUROPEAN PATENT SPECIFICATION

(11) **EP 4 427 769 B1**
(45) Date of publication and mention of the grant of the patent: **05.03.2025**
(21) Application number: 23160324.2
(22) Date of filing: 07.03.2023
(51) Int. Cl.: A61M 5/142, A61M 5/145, A61M 5/168, A61M 5/172, A61M 39/24

(54) **METERING ASSEMBLY FOR AN IMPLANTABLE INFUSION APPARATUS AND IMPLANTABLE INFUSION APPARATUS WITH SUCH A METERING ASSEMBLY**
DOSIERANORDNUNG FÜR EIN IMPLANTIERBARES INFUSIONSGERÄT UND IMPLANTIERBARES INFUSIONSGERÄT MIT EINER SOLCHEN DOSIERANORDNUNG
ENSEMBLE DE DOSAGE POUR UN APPAREIL DE PERFUSION IMPLANTABLE ET APPAREIL DE PERFUSION IMPLANTABLE DOTÉ D'UN TEL ENSEMBLE DE DOSAGE

(43) Date of publication of application: 11.09.2024
(73) Proprietor: B. Braun Miethke GmbH & Co. Kg, 14469 Potsdam (DE)
(72) Inventor: Ambrosio, Michel, 14974 Ludwigsfelde (DE); Loudermilk, Brandon, 14473 Potsdam (DE); Miethke, Christoph, 14469 Potsdam (DE); Böse, Gordon, 10317 Berlin (DE)
(74) Representative: Patentanwälte Ruff, Wilhelm, Beier, Dauster & Partner mbB

(56) References cited:
- WO-A1-2021/067674
- US-A- 5 049 141
- US-A1- 2011 060 318

## Description

### TECHNICAL FIELD AND PRIOR ART

This invention relates to a metering assembly according to the preamble of claim 1.

US patent number 5,049,141 discloses an implantable infusion apparatus comprising a metering assembly having the features of the preamble of claim 1. The prior art implantable infusion apparatus is intended for the delivery of an infusate, i.e., a medical fluid or a fluid drug, to a patient and comprises a pressurized infusate reservoir, an infusate dispensing member and said metering assembly. The infusate reservoir is filled with said infusate and pressurized at a pressure above the pressure inside the patient's body. The infusate dispensing member of the prior art apparatus is in the form of a catheter and configured to dispense infusate from the infusate reservoir to a site inside the patient's body. The metering assembly is configured to control the amount of infusate to be infused per unit of time, i.e., the drug dosage. For this purpose, the prior art metering assembly comprises an infusate accumulator with an accumulator volume and a pair of normally closed valves, i.e., an inlet valve and an outlet valve. The inlet valve controls the inlet of infusate from the reservoir into the accumulator volume. The outlet valve controls the outlet of infusate from the accumulator volume to the infusate dispensing member. In a first state, the inlet valve is open and the outlet valve is closed, thereby allowing an infusate flow from the pressurized infusate reservoir into the accumulator volume while blocking an infusate flow from the accumulator volume through the dispensing member. In a second state, the inlet valve is closed and the outlet valve is open, thereby allowing an infusate flow from the accumulator volume through the dispensing member while blocking an infusate flow from the pressurized infusate reservoir into the accumulator volume. The accumulator volume has a pressure intermediate between the pressure of the pressurized infusate reservoir and the pressure inside the patient's body. Therefore, in the second state, infusate accumulated inside the accumulator volume is "pumped" from the accumulator volume into the infusate dispensing member and thus into the patient's body. Cycling between the first and second state allows to control the infused drug dosage, wherein increasing the rate of cycling increases the infused volume per unit of time and thus the dosage of the drug. The prior art metering assembly comprises an actuator device configured for actuating the inlet valve and the outlet valve between said first and second states. The actuator device of the prior art metering assembly comprises two separate actuators for controlling the open and close cycles of the inlet valve and the outlet valve. Said separate actuators are in the form of electronically controlled solenoids, a first solenoid actuating the inlet valve and a second solenoid actuating the outlet valve. There is a potential risk that magnetic field effects, for example during a magnetic resonance imaging (MRI), cause a malfunction of the solenoids leading to an unintended opening of both the inlet valve and the outlet valve at the same time resulting in an uncontrolled drug overdose.

US 2011/060318 A1 discloses an implantable pump for providing metered doses of a compound to an individual. The implantable pump includes a chamber for storing an individual dose of the compound, an inlet valve fluidly coupled to the chamber for controlling a flow of the compound into the chamber, and an outlet valve fluidly coupled to the chamber for controlling a flow of the compound out of the chamber and into the individual. The pump includes an inlet valve driver circuit operable for opening and closing the inlet valve, and an outlet valve driver circuit operable for opening and closing the outlet valve. A battery and a charging circuit that includes a capacitor are included in the implantable pump. The charging circuit is operatively coupled to the battery for charging of the capacitor. The charged capacitor is operable for supplying power to the valve driver circuits.

WO 2021/067674 A1 discloses an implantable drug delivery device and a method that includes a sensor device for detecting deflection of a diaphragm at a first time from a first position within a diaphragm chamber of an accumulator of the implantable drug delivery device. A cycle count may be incremented in response to the diaphragm returning to the first position within the diaphragm chamber, each time during cyclical deflections within the diaphragm chamber. A flow rate of infusate may be determined based on a volume of the diaphragm chamber, the cycle count, and an elapsed time since the first time. Once the flow rate is determined, a processor may determine whether the flow rate of infusate is normal based on a comparison of the determined flow rate to at least one threshold flow rate.

### SUMMARY OF THE INVENTION

It is an object of this invention to provide a metering assembly as mentioned at the beginning, that has improved safety while being simple and robust in design.

This object is solved by providing the actuator device with an actuator movable between a first position and a second position for alternating actuation of the inlet valve or the outlet valve between the first state and the second state, wherein in the first position, the actuator mechanically actuates the inlet valve and does not actuate the outlet valve, and wherein in the second position, the actuator mechanically actuates the outlet valve and does not actuate the inlet valve. Due to the alternating mechanical actuation of both the inlet valve and the outlet valve by means of one and the same actuator, simultaneously actuating both valves is not possible. Consequently, opening the inlet valve and the outlet valve at the same time and a resulting uncontrolled infusate delivery is not possible. This leads to an improved safety. At the same time, using one and the same actuator for the actuation of both valves leads to a simple and robust design, allowing for an easy and inexpensive manufacture. In different embodiments, the actuator is differently movable between the first position and the second position. In one embodiment, the actuator moves linearly and/or in translation. In another embodiment, the actuator moves rotationally. In yet another embodiment, the actuator moves pivotally. Of course, a combined linear, rotational and/or pivotal motion of the actuator is also possible and envisaged. The movement between the first position and the second position can be the result of a rigid body motion of the actuator, an elastic deformation of at least a portion of the actuator or a combination of both. Both valves are normally closed, i.e., in a closed state blocking infusate flow if and when not actuated by means of the actuator. Consequently, the first position of the actuator controls the first state and the second position of the actuator controls the second state. In one embodiment, the inlet valve and the outlet valve each comprise a biasing member configured for biasing the respective valve into its closed position. In another embodiment, both valves each comprise a closing mechanism configured to keep the respective valve closed if and when not actuated. In different embodiments, the actuator actuates the valves in different ways. In one embodiment, the actuator is configured to open the valves by means of a pushing motion and/or a pushing force. In another embodiment, the actuator is configured to open the valves by means of a pulling motion and/or a pulling force. In yet another embodiment, the actuator is configured to open the valves by means of a turning motion and/or a torque. Of course, combinations thereof are possible and envisaged. The mechanical action of the actuator on the inlet valve and the outlet valve can be direct or indirect. In one embodiment, a portion of the actuator alternately directly contacts a portion of the inlet valve or a portion of the outlet valve, said contacts transmitting a force and/or motion of the actuator onto the respective valve in order to open it. In another embodiment, said alternating contact is indirect, the actuator acting on a transmitting member which in turn (directly) acts on the respective valve. The pressurized infusate reservoir and the infusate dispensing member do not form components of the metering assembly but instead of the implantable infusion apparatus. The infusate reservoir is configured to contain and pressurize the infusate by known techniques, for example by means of a two-phase fluid induced pressure. Technical details of the pressurized infusate reservoir are not essential to the present invention and therefore omitted. In a preferred embodiment, the infusate dispensing member is a catheter. However, technical details of the infusate dispensing member are not essential to the present invention and therefore omitted. The same applies, mutatis mutandis, regarding the infusate accumulator with the accumulator volume. In a preferred embodiment, the accumulator volume is formed by means of a diaphragm arranged in an accumulator chamber of the infusate accumulator, wherein said diaphragm is deflectable by means of the infusate inflow from the pressurized infusate reservoir while the valves reside in the first state. The pressure on a back side of the diaphragm can be said intermediate pressure causing the described "pumping" action of the infusate accumulator. However, other infusate accumulator designs are possible and envisaged.

In one embodiment, the actuator comprises an actuator member alternately acting on the inlet valve or the outlet valve, and a force generating member operatively coupled with the actuator member and configured for generating a force and/or motion for moving the actuator member between the first position and the second position. In one embodiment, the actuator member is a lever arm pivotally movable and/or flexibly bendable between the first position and the second position. In another embodiment, the actuator member is a push rod or a pull rod linearly movable between the first and second position. In yet another embodiment, the actuator member is a cam member rotationally movable between the first and second position. The force generating member is configured to generate a force and/or motion for moving the actuator member between the first and second position. In one embodiment, the force generating member is configured to move the actuator member from the first position into the second position, while moving the actuator member from the second position into the first position is not possible by means of the force generating member. In another embodiment, the force generating member is configured for moving the actuator member from the second position into the first position, while a reverse positioning is not possible by means of the force generating member. In these cases, the actuator preferably comprises a biasing member operatively coupled with the actuator member which biases the actuator member into the not actively actuated position. In yet another embodiment, the force generating member is configured to move the actuator member in both positions which allows to omit said biasing member. In a preferred embodiment, the force generating member is a piezoelectric element. In other embodiments, the force generating member relies on other physical operating principles, for example a hydraulic, pneumatic, magnetic principle to generate said force and/or motion.

In one embodiment, the actuator comprises a biasing member operatively coupled with the actuator member and biasing the actuator member against the force and/or motion of the force generating member. This enables the use of a single-direction force generating member which is configured to move the actuator member from the first position into the second position or vice versa but not alternately between both positions. Consequently, a further design simplification and cost reductions can be achieved. In one embodiment, the biasing member is a spring element, preferably in the form of a helical spring. In another embodiment, the biasing member is an elastomeric element or the like.

In one embodiment, the actuator member is a lever arm movable between the first position and the second position by means of the force generating member, the lever arm amplifying the motion of the force generating member into an amplified actuation motion. The lever arm leverages the motion of the force generating member. This allows to employ a force generating member having a relatively small range of motion/displacement which, without leveraging, would not suffice to actuate the valves.

Employing a small displacement/range of motion force generating member can lead to a further simplified design and reduced costs. The lever arm has a positive mechanical advantage regarding the motion of the force generating member. Preferably, said mechanical advantage is in a range between 1:5 and 1:60, preferably between 1:10 and 1:30, more preferably at least approximately 1:20. Consequently, due to the basic mechanical principles involved, the mechanical advantage on the force of the force generating member is negative.

In one embodiment, the lever arm is hinged at a pivot and pivotally movable between the first position and the second position. The pivot, for example, is in the form of a pin, a fulcrum or the like. In this embodiment, the lever arm is at least substantially rigid, the motion of the lever arm between the first and the second position, consequently, being a rigid body motion. This embodiment allows for a further simplified and yet more robust design.

In one embodiment, the lever arm is fixedly mounted and flexibly bendable between the first position and the second position. In this embodiment, at least a portion of the lever arm is elastic and/or pliable in order to allow a flexible bending motion between the first and the second position. This can be achieved by choosing appropriate elastic material and/or geometric properties of the lever arm. Since there is no need for a pivot and/or fulcrum, this embodiment allows for a very simple and very robust design.

In one embodiment, the inlet valve and the outlet valve are arranged at one end of the lever arm, said end of the lever arm alternately acting on both valves. This embodiment is space saving and allows for a compact design.

In one embodiment, the inlet valve and the outlet valve are arranged at opposing first and second ends of the lever arm, wherein the first end acts on the inlet valve and the second end acts on the outlet valve. This allows for additional degrees of freedom regarding the design of the metering assembly, in particular the arrangement of the inlet valve and the outlet valve with respect to other components. Moreover, it is possible to use different mechanical advantages for the actuation of the different valves. In this embodiment, the lever arm moves like a rocker or seesaw between the first position and the second position.

In one embodiment, the force generating member is a piezoelectric element, preferably in the form of a piezo stack. Piezoelectric elements are often referred to as piezoelectric actuators and work on the basis of known physical principles. Energizing the piezoelectric element causes the piezoelectric element to expand and/or extend. Deenergizing causes the piezoelectric element to return to its unexpanded/unextended (original) dimensions. Using a piezo stack, which is a known form of piezoelectric element, allows for a slender design while providing a relatively large range of motion/displacement of the force generating element. The piezoelectric element is a highly useful component as it has the ability to change shape in response to voltage. When a positive voltage is applied, it expands, whereas a negative voltage causes it to shrink. The degree of expansion varies depending on both the length of the piezoelectric element and the voltage applied. This means that the longer the piezoelectric element and the higher the voltage, the greater the expansion. Moreover, the material of the piezoelectric element generates a blocking force that relies on both the voltage and the cross-section of the piezoelectric element. A larger cross-section and higher voltage result in a greater blocking force that it can generate and transmit. The present invention takes advantage of the unique characteristics of the piezoelectric element/its material. In one embodiment its expansion is used and amplified using said lever arm to reach the desired amount of movement. This ensures operation within the required force's working range, while still achieving the desired displacement.

In one embodiment, the inlet valve and the outlet valve each comprise a movable ball valve element, a fixed valve seat and a biasing element, which biases the ball element into the valve seat, wherein the actuator at least indirectly acts on the ball valve element. This embodiment allows for a further simplified and very robust design. Preferably, the biasing element is a spring element, in particular a helical spring. The biasing element pushes the ball valve element into the valve seat, thereby biasing the respective valve into its closed position. In one embodiment, the actuator directly acts on the ball valve element for opening the respective valve. In another embodiment, said action is indirect via a transmitting member or the like.

In one embodiment, the inlet valve and the outlet valve each comprise an axially movable transmitting member longitudinally extending between a first end and a second end, wherein the first end of the transmitting member is configured for contacting the actuator and the second end of the transmitting member is configured for contacting the ball valve element. The transmitting member is configured to transmit the force and/or motion of the force generating member onto the respective valve, in particular onto the respective ball valve element. The transmitting member can have a single-part or a multi-part design. This embodiment allows to arrange the actuator further away from the valves and with this further away from fluid carrying components/portions of the metering assembly.

The invention further relates to an implantable infusion apparatus comprising a metering assembly according to the preceding disclosure, and further comprising a pressurized infusate reservoir and an infusate dispensing member, wherein the inlet conduit of the metering assembly is in fluid communication with the infusate reservoir, and wherein the outlet conduit of the metering assembly is in fluid communication with the infusate dispensing member.

### BRIEF DESCRIPTION OF THE DRAWINGS

In the following, exemplary embodiments of the invention are described in detail with reference to the drawings. Throughout the drawings, same elements are denoted with same reference numerals. The drawings schematically show:
- Fig. 1: an illustration of one embodiment of an implantable infusion apparatus according to the invention having one embodiment of a metering assembly according to the invention, the metering assembly being depicted in a cross-sectional view;
- Fig. 2: the metering assembly shown in Fig. 1 in a further sectional view with respect to a longitudinal section plane;
- Fig. 3: a cross-sectional view of a valve of the metering assembly according to Figs. 1 and 2;
- Fig. 4: a longitudinally sectioned view of a further embodiment of a metering device according to the invention; and
- Fig. 5: a further longitudinally sectioned view of the metering assembly according Fig. 4.

### DETAILED DESCRIPTION OF EXEMPLARY EMBODIMENTS OF THE INVENTION

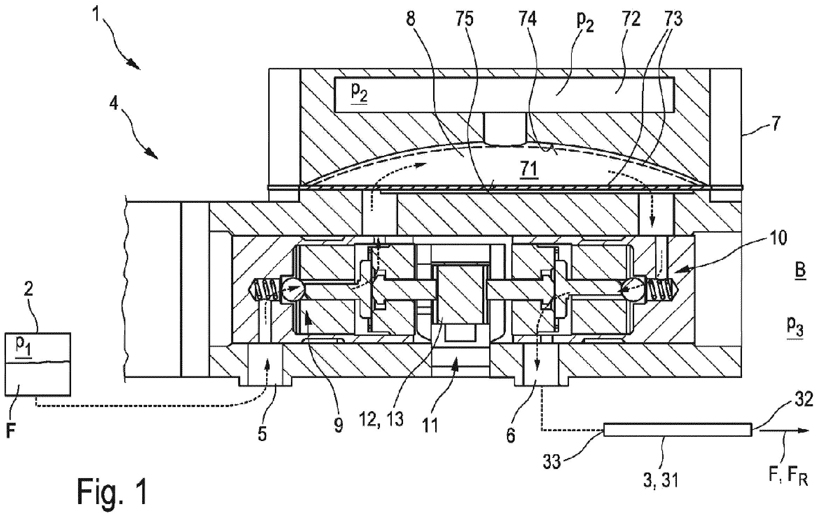

Referring to Fig. 1, an implantable infusion apparatus 1 comprises a pressurized infusate reservoir 2, an infusate dispensing member 3 and a metering assembly 4. The implantable infusion apparatus 1 is configured for the delivery of infusate F, i.e., a medical fluid or fluid drug, to a patient. In the configuration depicted in Fig. 1, the infusion apparatus 1 is implanted into the body B of the patient.

The infusate reservoir 2 contains a pressurized volume of the infusate F to be infused. Inside the infusate reservoir 2, the infusate F is pressurized with a reservoir pressure p₁. The reservoir pressure p₁ is generated by means of known techniques and can be constant or varying. Technical details of the pressurized infusate reservoir 2 are not within the focus of the present invention. A more detailed description of the specific design and function of the pressurized infusate reservoir 2 is therefore not necessary.

The infusate dispensing member 3 is configured for dispensing infusate F from the infusate reservoir 2 to a site inside the patient's body B. The infusate dispensing member 3 is fluidly connected with the infusate reservoir 2 via the metering assembly 4.

In the embodiment shown, the infusate dispensing member 3 is a catheter 31 having a distal end 32 and a proximal end 33. The distal end 32 can also be referred to as catheter tip and is located at said site inside the body B. The proximal end 33 is fluidly connected with the metering assembly 4.

In the embodiment shown, the pressurized infusate reservoir 2 and the metering assembly 4 are arranged in a housing which is not depicted in detail. The housing containing the infusate reservoir 2 and the metering assembly 4 is made of a biocompatible material and implanted into the patient's body B. In an embodiment not shown in the figures, the pressurized infusate reservoir and the metering assembly each comprise a separate housing, said housings being implanted at different locations of the patient's body.

The metering assembly 4 comprises an inlet conduit 5, an outlet conduit 6, an infusate accumulator 7 having an accumulator volume 8, an inlet valve 9, an outlet valve 10 and an actuator device 11.

The inlet conduit 5 is fluidly connected with the pressurized infusate reservoir 2. Said fluid connection is schematically depicted by means of the broken line extending between the infusate reservoir 2 and the inlet conduit 5 as shown in Fig. 1. The inlet conduit 5 is configured for receiving an infusate flow from the pressurized infusate reservoir 2.

The outlet conduit 6 is fluidly connected with the infusate dispensing member 3. Said fluid connection is schematically depicted by the broken line extending between the outlet conduit 6 and the fluid dispensing member 3 as shown in Fig. 1. The outlet conduit 6 is configured for delivering an infusate flow to the fluid dispensing member 3, i.e., the catheter 31. In the embodiment shown, the outlet conduit 6 is fluidly connected with the proximal end 33 of the fluid dispensing member 3 in form of the catheter 31.

The infusate accumulator 7 comprises the accumulator volume 8. The infusate accumulator 7 is configured for accumulating a defined fixed volume of infusate received from the infusate reservoir 2 and to be delivered to the infusate dispensing member 3. Said fixed volume is accumulated inside the accumulator volume 8. The accumulator volume 8 is in line with the inlet conduit 5 and the outlet conduit 6.

The inlet valve 9 is in fluid communication with the inlet conduit 5 and the accumulator volume 8. In other words, the inlet valve 9 is in line with the inlet conduit 5 and the accumulator volume 8. The inlet valve 9 controls the infusate flow from the infusate reservoir 2 into the accumulator volume 8.

The outlet valve 10 is in fluid communication with the accumulator volume 8 and the outlet conduit 6. In other words, the outlet valve 10 is in line with the accumulator volume 8 and the outlet conduit 6. The outlet valve 10 controls the infusate flow from the accumulator volume 8 into the fluid dispensing member 3.

The inlet valve 9 and the outlet valve 10 are both normally closed. This means that infusate flow through the valves 9, 10 is blocked if and when said valves 9, 10 are not actuated by means of the actuator device 11.

The actuator device 11 is configured for actuating the inlet valve 9 and the outlet valve 10 between a first state and a second state. In the first state, the inlet valve 9 is open and the outlet valve 10 is closed. The opened inlet valve 9 allows an infusate flow from the infusate reservoir 2 through the inlet conduit 5 into the accumulator volume 8. The closed outlet valve 10 simultaneously blocks an infusate flow from the accumulator volume 8 through the outlet conduit 6 and into the fluid dispensing member 3. In the second state, the inlet valve 9 is closed and the outlet valve 10 is open. The closed inlet valve 9 blocks an infusate flow from the infusate reservoir 2 through the inlet conduit 5 into the accumulator volume 8. Simultaneously, the opened outlet valve 10 allows an infusate flow from the accumulator volume 8 through the outlet conduit 6 into the fluid dispensing member 3.

Cycling or switching between first state and the second state causes a charging and discharging of the accumulator volume 8, i.e., a "pumping", whereby a rate of switching or cycling defines the amount of infusate F infused per unit of time and therefore the dosage of infusate F delivered to the patient. The amount of infusate F per unit of time can also be denoted as infusate rate F_{R}.

The aforementioned "pumping" action can be described in more detail as follows:
In a first step, the inlet valve 9 is open and the outlet valve 10 is closed, i.e., the valves 9, 10 are in the aforementioned first state. Consequently, the accumulator device 7 is filled to its fixed accumulator volume 8.

In a second step, the full accumulator volume 8 is discharged through the outlet conduit 6 by actuating the valves 9, 10 into the second state (inlet valve 9 closed, outlet valve 10 open).

Consequently, the accumulator device 7, more specifically its accumulator volume 8, is alternately filled and emptied by a switching action of the valves 9, 10.

In the embodiment shown, the accumulator device 7 operates on the basis of known principles and comprises a design which is known at least in principle. Hence, the accumulator device 7 operates at an accumulator pressure p₂ which is intermediate between the reservoir pressure p₁ and a pressure p₃ inside the patient's body B. The pressure p₃ inside the patient's body B may be an atmospheric or cardiovascular pressure slightly above the atmospheric pressure. Said pressure setup, i.e., p₁ > p₂ > p₃, results in the above-described pumping action.

In the specific embodiment shown, the accumulator device 7 comprises an accumulator chamber 71, a pressure chamber 72, a diaphragm 73, a back stop surface 74 and a front stop surface 75.

It is worth stressing, that the depicted design of the accumulator device is optional and not essential to the present invention. Consequently, embodiments not shown in the figures, comprise differently designed accumulator devices.

Now with further reference to the specific design of the accumulator device 7 of the shown embodiment, the diaphragm 73 is arranged in the accumulator chamber 71. The diaphragm 73 is deflectable between a first position as depicted in Fig. 1 and a second position. The second position of the diaphragm 73 is schematically illustrated with the broken line inside the accumulator chamber shown in Fig. 1. In its first position, the diaphragm 73 lays flat on the front stop surface 75. In its second position, the diaphragm 73 is deflected from the front stop surface 75 and pressed onto the back stop surface 74, thereby defining said fixed accumulator volume 8 inside the accumulator chamber 71. The pressure chamber 72 is pressurized at the accumulator pressure p₂ and in fluid communication with a back surface of the diaphragm 73 which opposes the accumulator volume 8. It is worth mentioning, that embodiments not shown in the figures, do not comprise a pressure chamber 72.

Now referring again to the above-mentioned steps of the pumping action, the accumulator volume 8 is empty when the diaphragm 73 is in its first position. Opening the inlet valve 9 while keeping the outlet valve 10 closed, causes the inflowing infusate to deflect the diaphragm 73 into its second position, thereby filling accumulator chamber 71 and/or the accumulator volume 8 to its defined fixed volume. Then, closing the inlet valve 9 and opening the outlet valve 10, i.e., actuating the valves 9, 10 into their second state, causes the accumulator pressure p₂ to press the diaphragm 73 back into its first position flat against the front stop surface 75, thereby emptying and discharging the accumulator volume 8 through the outlet conduit 6.

Since p₁ > p₃, opening both valves 9, 10 at the same time would cause an uncontrolled infusate flow from the infusate reservoir 2 through the metering assembly 4, from there directly into the fluid dispensing member 3 and further into the patient's body B. This would lead to an uncontrolled delivery of infusate F to the patient.

In order to avoid the risk of such an uncontrolled infusate delivery, the actuator device 11 comprises an actuator 12 which is movable between a first position and a second position for alternating actuation of the inlet valve 9 or the outlet valve 10 between the first state and the second state, wherein in the first position, the actuator 12 actuates the inlet valve 9 and does not actuate the outlet valve 10, and wherein in the second position, the actuator 12 actuates the outlet valve 10 and does not actuate the inlet valve 9.

Since both valves 9, 10 are normally closed and alternately actuated by one and the same actuator 12, it is not possible to simultaneously open both valves 9, 10 by means of the actuator 12. The alternating actuation of both valves 9, 10 by means of one and the same actuator 12 leads to an improved safety and simple design.

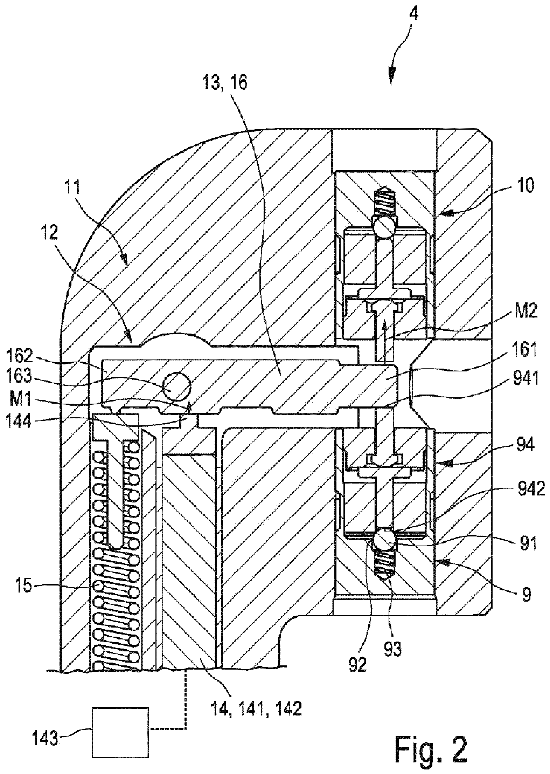

Referring to Figs. 1 and 2, said first position of the actuator 12 is depicted. Since both valves 9, 10 are normally closed, the first position of the actuator 12 controls the first state of the valves 9, 10 and the second position of the actuator 12 controls the second state of the valves 9, 10. In other words, moving the actuator 12 to from its second position into its first position opens the inlet valve 9 and deactivates the actuation of the outlet valve 10 causing it to close. Moving the actuator 12 from its first position into its second position deactivates the actuation of the inlet valve 9 causing it to close, while the outlet valve 10 is actuated and thereby open.

Referring now to Fig. 2, the actuator 12 comprises an actuator member 13 and a force generating member 14. The actuator member 13 is configured to directly or indirectly act - by means of a force and/or motion, i.e., mechanically - on the valves 9, 10. The force generating member 14 is configured to generate a force and/or motion for moving the actuator member 13 between its first and second position. It is worth mentioning that such a design of the actuator 12 is optional. Hence, in embodiments not shown in the figures, the functions of force and/or motion generation and the function of acting on the valves are integrated into one and the same member.

In the embodiment shown, the actuator 12 further comprises a biasing member 15. The biasing member 15 is configured for biasing the actuator member 12 against the force and/or motion of the force generating member 14. The biasing member 15 is optional and, in particular, not needed in case the force generating member 14 is configured to move the actuator member 13 back and forth between the first position and the second position. In the embodiment shown, however, the force generating member 14 is configured to move the actuator member 12 from its first position into its second position - but not vice versa - and against the bias of the biasing member 15. In the embodiment shown, moving the actuator member 13 from its second position into its first position solely by means of the force generating member 14 is not possible. Said motion of the actuator member 13 is instead induced by means of the biasing member 15. Of course, other embodiments are envisaged where the force generating member is configured to move the actuator member from its second to its first position but not the other way around.

In the embodiment shown, the actuator member 13 is a lever arm 16. The lever arm 16 is movable between the first position and the second position by means of the force generating member 14, thereby amplifying the motion of the force generating member 14 into an amplified actuation motion acting on the inlet valve 9 or, respectively, the outlet valve 10.

The lever arm 16 longitudinally extends between a first end 161 and a second end 162 (see Fig. 2). In the embodiment shown in Figs. 1 and 2, the first end 161 alternately acts on the inlet valve 9 or the outlet valve 10.

In the embodiment shown, the lever arm 16 is hinged at a pivot 163 and, consequently, pivotally movable between its first position and its second position. The pivot 163 can also be referred to as fulcrum.

In the embodiment shown, the biasing member 15 is arranged at the second end 162 of the lever arm 16. The force generating member 14 is located between the pivot 163 and the first end 161 of the lever arm 16. The distance between the first end 161 acting on the valves 9, 10 and the pivot 163 as well as the distance between the force generating member 14 and the pivot 163 defines the amplification, i.e., leverage and/or mechanical advantage, of the lever arm 16.

In the embodiment shown, a motion M1 generated by means of the force generating member 14 is amplified into an actuation motion M2 in a ratio of 1:20.

The described mechanical leverage principles apply, mutatis mutandis, also with respect to the bias generated by means of the biasing member 15.

In embodiments not shown in the figures, the force generating member is located, for example, at the second end of the lever arm, while the biasing member is located, for example, between the pivot and the first end. In other words, the specific arrangement of the force generating member 14, the biasing member 15 and the pivot 163 with respect to each other and to the first end 161 and the second end 162 as depicted in Fig. 2 is exemplary and may be different in other embodiments.

Further, with reference to an embodiment not shown in the figures, the lever arm is fixedly mounted and flexibly bendable between the first position and the second position. Hence, the pivot 163 shown in Fig. 2 is optional.

In the embodiment shown, the force generating member 14 is a piezoelectric element 141. The piezoelectric element 141 is, in the shown embodiment, in the form of a piezo stack 142, i.e., a longitudinally shaped piezoelectric element, while other shapes and forms are possible as well.

The piezoelectric element 141 operates on the basis of known physical principles. In particular, the piezoelectric element 141 extends and/or expands when subjected to electrical energy. When deenergized, the piezoelectric element 141 returns into its original unexpanded/unextended state.

In the embodiment shown, energizing and deenergizing of the piezoelectric element 141 is controlled by means of a control unit 143. Said control unit 143 can be provided as an integral component of the metering assembly 4. Alternatively, the control unit 143 can be provided as a separate component connected to the metering assembly 4, in particular the piezoelectric element 141. In Fig. 2, the control unit 143 is schematically depicted, wherein said connection to the piezoelectric element 141 is symbolized by means of a broken line. In an embodiment not shown in the figures, the control unit is not part of the apparatus 1 but instead a separate unit external from the patient's body B and connected to the apparatus by means of a suitable wireless connection. Consequently, the control unit 143 is optional and not essential to the present invention.

In the embodiment shown, energizing the piezoelectric element 141 causes one end 144 of the piezoelectric element 141 to move and press against the lever arm 16. This causes the lever arm 16 to pivotally move around the pivot 163 - with respect to Fig. 2 - in a counter-clockwise direction, whereby its first end 161 acts on the outlet valve 10. In its energized state, the piezoelectric element 141 works against the bias of the biasing member 15. Deenergizing the piezoelectric element 141 causes the end 144 to move away from the lever arm 16 and, consequently, giving way to a clockwise pivotal movement of the lever arm 16 induced by the bias of the biasing member 15. The first end 161, consequently, moves away from the outlet valve 10 deactivating its actuations and instead actuates the inlet valve 9.

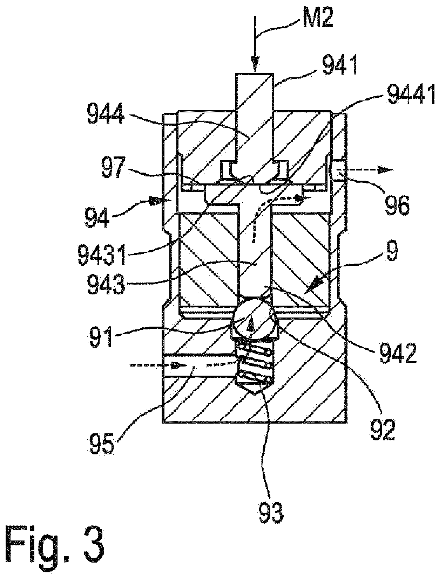

In the embodiment shown, the inlet valve 9 and the outlet valve 10 comprise a specific design which is illustrated by means of Fig. 3 and with respect to the inlet valve 9. The inlet valve 9 and the outlet valve 10 are identical. Hence, the following description of details of the inlet valve 9 applies, mutatis mutandis, also with respect to the outlet valve 10.

Now referring to Fig. 3, the inlet valve 9 comprises a movable ball valve element 91, a fixed valve seat 92 and a biasing element 93. The biasing element 93 biases the ball valve element 91 into the valve seat 92, thereby closing the inlet valve 9 and thus causing the normally closed state of the inlet valve 9. The actuator 12 and more specifically the actuator member 13 in form of the lever arm 16 (not depicted in Fig. 3) acts on the ball valve element 91 and against the bias of the biasing element 93.

In the embodiment shown, the actuator 12 acts indirectly on the ball valve element 91 by means of a transmitting member 94 of the inlet valve 9. The actuation motion M2 of the actuator 12 is transmitted onto the ball valve element 91 by means of said transmitting member 94 in order to open the inlet valve 9.

In the embodiment shown, both valves 9, 10 further comprise a valve inlet passage 95 and a valve outlet passage 96. Regarding the inlet valve 9, the valve inlet passage 95 is fluidly connected with the inlet conduit 5 while the valve outlet passage 96 is in fluid connection with the accumulator volume 8. Now regarding the outlet valve 10, its valve inlet passage 95 is in fluid connection with the accumulator volume 8 while its valve outlet passage 96 is in fluid connection with the outlet conduit 6.

The ball valve element 91 and the valve seat 92 are in line with the valve inlet passage 95 and the valve outlet passage 96. Moving the ball valve element 91 away from the valve seat 92 allows an infusate flow between the valve inlet passage 95 and the valve outlet passage 96.

The transmitting member 94 longitudinally extends between a first end 941 and a second end 942. The first end 941 is configured for being contacted by the actuator 12 or more specifically with respect to the shown embodiment, the first end 161 of the lever arm 16. The second end 942 of the transmitting member 94 is configured for contacting the ball valve element 91. The transmitting member 94 is axially movable by means of the lever arm 16 in order to open the valve 9. More specifically, moving the lever arm 16 in its first position causes the first end 161 of the lever arm 16 to press onto the first end 941 of the transmitting member 94, whereby the actuation motion M2 is transmitted onto the ball valve element 91, moving the ball valve element 91 away from the valve seat 92 and thus oping the inlet valve 9. Moving the lever arm 16 back in its second position causes the bias of the biasing element 93 to move the ball valve element 91 back and into the valve seat 92, thereby also moving the transmitting member 94 back to its original position.

In the embodiment shown, the transmitting member 94 has a two-part design comprising a first transmitting pin 943 and a second transmitting pin 944. Said pins 943, 944 are coaxial to each other and to the ball valve element 91. The first transmitting pin 943 comprises the second end 942 and is located in line with the valve seat 92 and the valve outlet passage 96. The first transmitting pin 943 is consequently located in a fluid carrying region of the valve 9. The second pin 944, in contrast, is located in a "dry" region of the valve 9 which is not fluid carrying. The second transmitting pin 944 comprises the first end 941 of the transmitting member 94 and a contact end 9441. Said contact end 9441 opposes the first end 941 and contacts a complementary contact end 9431 of the first transmitting pin 943 via a fluid-tight flexible septum 97 which fluidly separates said fluid-carrying and dry regions of the inlet valve 9.

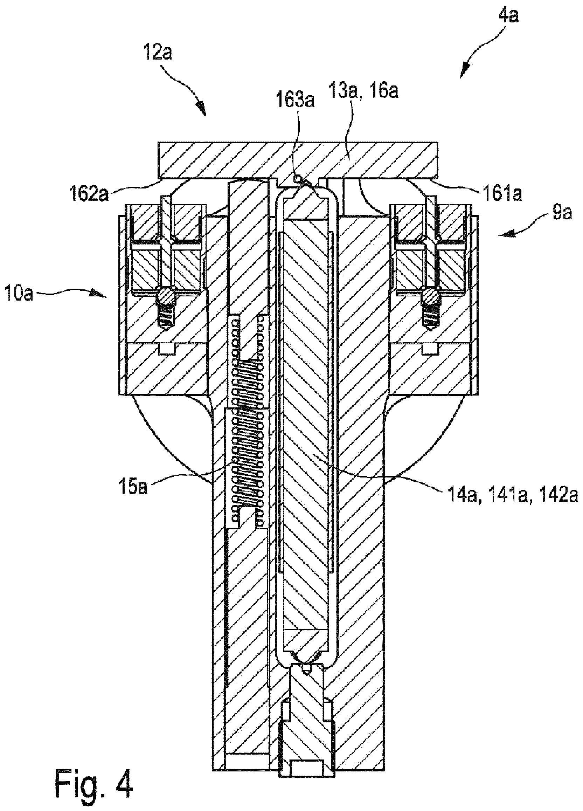

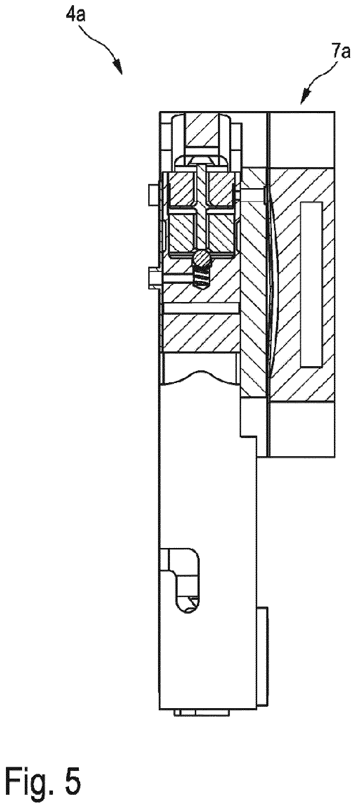

Figs. 4 and 5 illustrate a further embodiment of a metering assembly 4a according to the invention. The metering assembly 4a is substantially identical to the metering assembly 4 of the embodiment according to Figs. 1 to 3. To avoid repetition, only relevant differences of the metering assembly 4a over the metering assembly 4 will be described. If not described differently, the disclosure regarding the metering assembly 4 also applies, mutatis mutandis, with respect to the metering assembly 4a. Hence, not all features/details of the metering assembly 4a are described separately. Instead, reference is made to the description of the metering assembly 4.

The metering assembly 4a according to Figs. 4 and 5 differs in that the inlet valve 9a and the outlet valve 10a are arranged at the opposing ends 161a, 162a of the lever arm 16a. The inlet valve 9a is arranged at the first end 161a. The outlet valve 10a is arranged at the second end 162a. The first end 161a is configured to actuate the inlet valve 9a while the second end 162a is configured to actuate the outlet valve 10a. The lever arm 16a is hinged at a pivot 163a and, consequently, pivotally movable between its first position, in which the first end 161a actuates an opening of the inlet valve 9a, and its second position, in which the second end 162a actuates an opening of the outlet valve 10a. The pivot 163a is located substantially in the middle between the first end 161a and the second end 162a. The force generating member 14a acts on a contact point (without reference sign) longitudinally located between the first end 161a and the pivot 163a, thereby forming a T-shape with the lever arm 16a. The biasing member 15a contacts the lever arm 16a at a contact point (without reference sign) located longitudinally between the second end 162a and the pivot 163a.

## Claims

1. A metering assembly (4, 4a) for an implantable infusion apparatus (1) comprising:
an inlet conduit (5) configured for fluid connection with a pressurized infusate reservoir (2) of the infusion apparatus (1);
an outlet conduit (6) configured for fluid connection with an infusate dispensing member (3) of the infusion apparatus (1);
an infusate accumulator (7, 7a) having an accumulator volume (8);
an inlet valve (9, 9a) in fluid communication with the inlet conduit (5) and the accumulator volume (8);
an outlet valve (10, 10a) in fluid communication with the accumulator volume (8) and the outlet conduit (6);
wherein the inlet valve (9, 9a) and the outlet valve (10, 10a) are both normally closed; and
an actuator device (11) configured for actuating the inlet valve (9, 9a) and the outlet valve (10, 10a) between a first state and a second state,
wherein in the first state, the inlet valve (9, 9a) is open and the outlet valve (10, 10a) is closed, thereby allowing an infusate flow through the inlet conduit (5) into the accumulator volume (8) while blocking an infusate flow from the accumulator volume (8) through the outlet conduit (6), and
wherein in the second state, the inlet valve (9, 9a) is closed and the outlet valve (10, 10a) is open, thereby allowing an infusate flow from the accumulator volume (8) through the outlet conduit (6) while blocking an infusate flow through the inlet conduit (5) into the accumulator volume (8);
**characterized in that**
the actuator device (11) comprises an actuator (12, 12a) movable between a first position and a second position for alternating actuation of the inlet valve (9, 9a) and the outlet valve (10, 10a) between the first state and the second state,
wherein in the first position, the actuator (12, 12a) mechanically actuates the inlet valve (9, 9a) and does not actuate the outlet valve (10, 10a), and
wherein in the second position, the actuator (12, 12a) mechanically actuates the outlet valve (10, 10a) and does not actuate the inlet valve (9, 9a).

2. The metering assembly (4, 4a) of claim 1, wherein the actuator (12, 12a) comprises an actuator member (13, 13a) alternately acting on the inlet valve (9, 9a) and
the outlet valve (10, 10a), and a force generating member (14, 14a) operatively coupled with the actuator member (13, 13a) and configured for generating a force and/or motion for moving the actuator member (13, 13a) between the first position and the second position.

3. The metering assembly (4, 4a) of claim 2, wherein the actuator (12, 12a) comprises a biasing member (15, 15a) operatively coupled with the actuator member (13, 13a) and biasing the actuator member (13, 13a) against the force and/or motion of the force generating member (14, 14a).

4. The metering assembly (4, 4a) of claim 2 or 3, wherein the actuator member (13, 13a) is a lever arm (16, 16a) movable between the first position and the second position by means of the force generating member (14, 14a), the lever arm (16, 16a) amplifying the motion of the force generating member (14, 14a) into an amplified actuation motion.

5. The metering assembly (4, 4a) of claim 4, wherein the lever arm (16, 16a) is hinged at a pivot (163, 163a) and pivotally movable between the first position and the second position.

6. The metering assembly of claim 4, wherein the lever arm is fixedly mounted and flexibly bendable between the first position and the second position.

7. The metering assembly (4, 4a) according to one of the claims 4 to 6, wherein the inlet valve (9) and the outlet valve (10) are arranged at one end (161) of the lever arm (16), said end (161) of the lever arm (16) alternately acting on both valves (9, 10).

8. The metering assembly (4a) according to any of claims 4 to 6, wherein the inlet valve (9a) and the outlet valve (10a) are arranged at opposing first and second ends (161a, 162a) of the lever arm (16a), wherein the first end (161a) acts on the inlet valve (9a) and the second end (162a) acts on the outlet valve (10a).

9. The metering assembly (4, 4a) according to any of claims 2 to 8, wherein the force generating member (14, 14a) is a piezoelectric element (141, 141a), preferably in the form of a piezo stack (142, 142a).

10. The metering assembly (4, 4a) according to any of the preceding claims, wherein the inlet valve (9, 9a) and the outlet valve (10, 10a) each comprise a movable ball valve element (91), a fixed valve seat (92) and a biasing element (93), which biases the ball valve element (91) into the valve seat (92), wherein the actuator (12, 12a) at least indirectly acts on the ball valve element (91).

11. The metering assembly (4, 4a) according to claim 10, wherein the inlet valve (9, 9a) and the outlet valve (10, 10a) each comprise an axially movable transmitting member (94) longitudinally extending between a first end (941) and a second end (942), wherein the first end (941) of the transmitting member (94) is configured for contacting the actuator (12, 12a) and the second end (942) of the transmitting member (94) is configured for contacting the ball valve element (91).

12. An implantable infusion apparatus (1) for the delivery of infusate (F) to a patient comprising a metering assembly (4, 4a) according to any of the preceding claims, and further comprising a pressurized infusate reservoir (2) and an infusate dispensing member (3), wherein the inlet conduit (5) of the metering assembly (4, 4a) is in fluid communication with the infusate reservoir (2), and wherein the outlet conduit (6) of the metering assembly (4, 4a) is in fluid communication with the infusate dispensing member (3).

## Patentansprüche

1. Dosieranordnung (4, 4a) für ein implantierbares Infusionsgerät (1), umfassend:
- eine Einlassleitung (5), die für eine Fluidverbindung mit einem unter Druck stehenden Infusatreservoir (2) des Infusionsgeräts (1) konfiguriert ist;
- eine Auslassleitung (6), die für eine Fluidverbindung mit einem Infusatabgabeteil (3) des Infusionsgeräts (1) konfiguriert ist;
- einen Infusat-Akkumulator (7, 7a) mit einem Akkumulatorvolumen (8);
- ein Einlassventil (9, 9a) in Fluidverbindung mit der Einlassleitung (5) und dem Akkumulatorvolumen (8);
- ein Auslassventil (10, 10a) in Fluidverbindung mit dem Akkumulatorvolumen (8) und der Auslassleitung (6);
- wobei das Einlassventil (9, 9a) und das Auslassventil (10, 10a) beide normalerweise geschlossen sind; und
- eine Betätigungsvorrichtung (11), die zum Betätigen des Einlassventils (9, 9a) und des Auslassventils (10, 10a) zwischen einem ersten Zustand und einem zweiten Zustand konfiguriert ist,
- wobei im ersten Zustand das Einlassventil (9, 9a) offen ist und das Auslassventil (10, 10a) geschlossen ist, wodurch ein Infusatfluss durch die Einlassleitung (5) in das Akkumulatorvolumen (8) ermöglicht wird, während ein Infusatfluss aus dem Akkumulatorvolumen (8) durch die Auslassleitung (6) blockiert wird, und
- wobei im zweiten Zustand das Einlassventil (9, 9a) geschlossen ist und das Auslassventil (10, 10a) offen ist, wodurch ein Infusatfluss vom Akkumulatorvolumen (8) durch die Auslassleitung (6) ermöglicht wird, während ein Infusatfluss durch die Einlassleitung (5) in das Akkumulatorvolumen (8) blockiert wird;
**dadurch gekennzeichnet, dass**
- die Betätigungsvorrichtung (11) einen Aktuator (12, 12a) umfasst, der zwischen einer ersten Position und einer zweiten Position beweglich ist, um das Einlassventil (9, 9a) und das Auslassventil (10, 10a) zwischen dem ersten Zustand und dem zweiten Zustand abwechselnd zu betätigen,
- wobei der Aktuator (12, 12a) in der ersten Position das Einlassventil (9, 9a) mechanisch betätigt und das Auslassventil (10, 10a) nicht betätigt, und
- wobei in der zweiten Position der Aktuator (12, 12a) das Auslassventil (10, 10a) mechanisch betätigt und das Einlassventil (9, 9a) nicht betätigt.

2. Dosieranordnung (4, 4a) nach Anspruch 1, wobei der Aktuator (12, 12a) ein Betätigungselement (13, 13a), das abwechselnd auf das Einlassventil (9, 9a) und das Auslassventil (10, 10 a) wirkt, und ein Krafterzeugungselement (14, 14a) umfasst, das mit dem Betätigungselement (13, 13a) wirkverbunden ist und zum Erzeugen einer Kraft und/oder Bewegung konfiguriert ist, um das Betätigungselement (13, 13a) zwischen der ersten Position und der zweiten Position zu bewegen.

3. Dosieranordnung (4, 4a) nach Anspruch 2, wobei der Aktuator (12, 12a) ein Vorspannelement (15, 15a) umfasst, das mit dem Betätigungselement (13, 13a) wirkverbunden ist und das Betätigungselement (13, 13a) gegen die Kraft und/oder die Bewegung des Krafterzeugungselements (14, 14a) vorspannt.

4. Dosieranordnung (4, 4a) nach Anspruch 2 oder 3, wobei das Betätigungselement (13, 13a) ein Hebelarm (16, 16a) ist, der mittels des Krafterzeugungselements (14, 14a) zwischen der ersten Position und der zweiten Position bewegbar ist, wobei der Hebelarm (16, 16a) die Bewegung des Krafterzeugungselements (14, 14a) in eine verstärkte Betätigungsbewegung verstärkt.

5. Dosieranordnung (4, 4a) nach Anspruch 4, wobei der Hebelarm (16, 16a) an einem Drehgelenk (163, 163a) angelenkt ist und zwischen der ersten Position und der zweiten Position schwenkbar ist.

6. Dosieranordnung nach Anspruch 4, wobei der Hebelarm fest montiert ist und zwischen der ersten Position und der zweiten Position flexibel biegbar ist.

7. Dosieranordnung (4, 4a) nach einem der Ansprüche 4 bis 6, wobei das Einlassventil (9) und das Auslassventil (10) an einem Ende (161) des Hebelarms (16) angeordnet sind, wobei das Ende (161) des Hebelarms (16) abwechselnd auf beide Ventile (9, 10) einwirkt.

8. Dosieranordnung (4a) nach einem der Ansprüche 4 bis 6, wobei das Einlassventil (9a) und das Auslassventil (10a) an gegenüberliegenden ersten und zweiten Enden (161a, 162a) des Hebelarms (16a) angeordnet sind, wobei das erste Ende (161a) auf das Einlassventil (9a) und das zweite Ende (162a) auf das Auslassventil (10a) wirkt.

9. Dosieranordnung (4, 4a) nach einem der Ansprüche 2 bis 8, wobei das Krafterzeugungselement (14, 14a) ein piezoelektrisches Element (141, 141a) ist, vorzugsweise in Form eines Piezostapels (142, 142a).

10. Dosieranordnung (4, 4a) nach einem der vorhergehenden Ansprüche, wobei das Einlassventil (9, 9a) und das Auslassventil (10, 10a) jeweils ein bewegliches Kugelventilelement (91), einen festen Ventilsitz (92) und ein Vorspannelement (93) umfassen, welches das Kugelventilelement (91) in den Ventilsitz (92) vorspannt, wobei der Aktuator (12, 12a) zumindest indirekt auf das Kugelventilelement (91) wirkt.

11. Dosieranordnung (4, 4a) nach Anspruch 10, wobei das Einlassventil (9, 9a) und das Auslassventil (10, 10a) jeweils ein axial bewegliches Übertragungselement (94) umfassen, das sich in Längsrichtung zwischen einem ersten Ende (941) und einem zweiten Ende (942) erstreckt, wobei das erste Ende (941) des Übertragungselements (94) derart konfiguriert ist, dass es den Aktuator (12, 12a) berührt, und das zweite Ende (942) des Übertragungselements (94) derart konfiguriert ist, dass es das Kugelventilelement (91) berührt.

12. Implantierbares Infusionsgerät (1) zur Abgabe von Infusat (F) an einen Patienten, umfassend eine Dosieranordnung (4, 4a) gemäß einem der vorhergehenden Ansprüche und ferner umfassend ein unter Druck stehendes Infusatreservoir (2) und ein Infusatabgabeteil (3), wobei die Einlassleitung (5) der Dosieranordnung (4, 4a) in Fluidverbindung mit dem Infusatreservoir (2) steht und wobei die Auslassleitung (6) der Dosieranordnung (4, 4a) in Fluidverbindung mit dem Infusatabgabeteil (3) steht.

## Revendications

1. Ensemble de dosage (4, 4a) pour un appareil de perfusion implantable (1) comprenant :
un conduit d'entrée (5) conçu pour un raccordement fluidique avec un réservoir de solution intraveineuse sous pression (2) de l'appareil de perfusion (1) ;
un conduit de sortie (6) conçu pour un raccordement fluidique avec un élément de distribution de solution intraveineuse (3) de l'appareil de perfusion (1) ;
un accumulateur de solution intraveineuse (7, 7a) comportant un volume d'accumulateur (8) ;
une valve d'entrée (9, 9a) en communication fluidique avec le conduit d'entrée (5) et le volume d'accumulateur (8) ;
une valve de sortie (10, 10a) en communication fluidique avec le volume d'accumulateur (8) et le conduit de sortie (6) ;
la valve d'entrée (9, 9a) et la valve de sortie (10, 10a) étant toutes deux normalement fermées ; et
un dispositif actionneur (11) conçu pour actionner la valve d'entrée (9, 9a) et la valve de sortie (10, 10a) entre un premier état et un second état,
dans le premier état, la valve d'entrée (9, 9a) étant ouverte et la valve de sortie (10, 10a) étant fermée,
permettant ainsi un écoulement de solution intraveineuse à travers le conduit d'entrée (5) dans le volume d'accumulateur (8) tout en bloquant un écoulement de solution intraveineuse depuis le volume d'accumulateur (8) à travers le conduit de sortie (6), et
dans le second état, la valve d'entrée (9, 9a) étant fermée et la valve de sortie (10, 10a) étant ouverte,
permettant ainsi un écoulement de solution intraveineuse depuis le volume d'accumulateur (8) à travers le conduit de sortie (6) tout en bloquant l'écoulement de solution intraveineuse à travers le conduit d'entrée (5) dans le volume d'accumulateur (8) ;
**caractérisé en ce que**
le dispositif actionneur (11) comprend un actionneur (12, 12a) mobile entre une première position et une seconde position pour actionner alternativement la valve d'entrée (9, 9a) et la valve de sortie (10, 10a) entre le premier état et le second état,
dans la première position, l'actionneur (12, 12a) actionnant mécaniquement la valve d'entrée (9, 9a) et
n'actionnant pas la valve de sortie (10, 10a), et
dans la seconde position, l'actionneur (12, 12a) actionnant mécaniquement la valve de sortie (10, 10a) et
n'actionnant pas la valve d'entrée (9, 9a).

2. Ensemble de dosage (4, 4a) selon la revendication 1, l'actionneur (12, 12a) comprenant un élément actionneur (13, 13a) agissant alternativement sur la valve d'entrée (9, 9a) et la valve de sortie (10, 10a), et un élément générateur de force (14, 14a) accouplé fonctionnellement à l'élément actionneur (13, 13a) et conçu pour générer une force et/ou un mouvement pour déplacer l'élément actionneur (13, 13a) entre la première position et la seconde position.

3. Ensemble de dosage (4, 4a) selon la revendication 2, l'actionneur (12, 12a) comprenant un élément de sollicitation (15, 15a) accouplé fonctionnellement à l'élément actionneur (13, 13a) et sollicitant l'élément actionneur (13, 13a) contre la force et/ou le mouvement de l'élément générateur de force (14, 14a).

4. Ensemble de dosage (4, 4a) selon la revendication 2 ou 3, l'élément actionneur (13, 13a) étant un bras de levier (16, 16a) mobile entre la première position et la seconde position au moyen de l'élément générateur de force (14, 14a), le bras de levier (16, 16a) amplifiant le mouvement de l'élément générateur de force (14, 14a) en un mouvement d'actionnement amplifié.

5. Ensemble de dosage (4, 4a) selon la revendication 4, le bras de levier (16, 16a) étant articulé au niveau d'un pivot (163, 163a) et mobile de manière pivotante entre la première position et la seconde position.

6. Ensemble de dosage selon la revendication 4, le bras de levier étant monté à demeure et pouvant être plié de manière flexible entre la première position et la seconde position.

7. Ensemble de dosage (4, 4a) selon l'une quelconque des revendications 4 à 6, la valve d'entrée (9) et la valve de sortie (10) étant disposées au niveau d'une extrémité (161) du bras de levier (16), ladite extrémité (161) du bras de levier (16) agissant alternativement sur les deux valves (9, 10).

8. Ensemble de dosage (4a) selon l'une quelconque des revendications 4 à 6, la valve d'entrée (9a) et la valve de sortie (10a) étant disposées au niveau de première et seconde extrémités (161a, 162a) opposées du bras de levier (16a), la première extrémité (161a) agissant sur la valve d'entrée (9a) et la seconde extrémité (162a) agissant sur la valve de sortie (10a).

9. Ensemble de dosage (4, 4a) selon l'une quelconque des revendications 2 à 8, l'élément générateur de force (14, 14a) étant un élément piézoélectrique (141, 141a), de préférence sous la forme d'une pile piézoélectrique (142, 142a).

10. Ensemble de dosage (4, 4a) selon l'une quelconque des revendications précédentes, la valve d'entrée (9, 9a) et la valve de sortie (10, 10a) comprenant chacune un élément robinet à boisseau sphérique (91) mobile, un siège de valve fixe (92) et un élément de sollicitation (93), qui sollicite l'élément robinet à boisseau sphérique (91) dans le siège de valve (92), l'actionneur (12, 12a) agissant au moins indirectement sur l'élément robinet à boisseau sphérique (91).

11. Ensemble de dosage (4, 4a) selon la revendication 10, la valve d'entrée (9, 9a) et la valve de sortie (10, 10a) comprenant chacune un élément de transmission (94) axialement mobile s'étendant longitudinalement entre une première extrémité (941) et une seconde extrémité (942), la première extrémité (941) de l'élément de transmission (94) étant conçue pour entrer en contact avec l'actionneur (12, 12a) et la seconde extrémité (942) de l'élément de transmission (94) étant conçue pour entrer en contact avec l'élément robinet à boisseau sphérique (91).

12. Appareil de perfusion implantable (1) pour l'administration d'une solution intraveineuse (F) à un patient, comprenant un ensemble de dosage (4, 4a) selon l'une quelconque des revendications précédentes, et comprenant en outre un réservoir de solution intraveineuse sous pression (2) et un élément de distribution de solution intraveineuse (3), le conduit d'entrée (5) de l'ensemble de dosage (4, 4a) étant en communication fluidique avec le réservoir de solution intraveineuse (2), et le conduit de sortie (6) de l'ensemble de dosage (4, 4a) étant en communication fluidique avec l'élément de distribution de solution intraveineuse (3).
